# EUROPEAN PATENT APPLICATION

(11) **EP 3 705 892 A1**
(43) Date of publication of application: **09.09.2020**
(21) Application number: 20161512.7
(22) Date of filing: 06.03.2020
(51) Int. Cl.: G01N 33/543, G01N 33/574

(54) **DETECTION METHOD AND DETECTION DEVICE OF CIRCULATING TUMOR CELL**

(30) Priority: 07.03.2019 JP 2019042025
(71) Applicant: Showa University, Tokyo 142-8555 (JP)
(72) Inventor: NAOE, Michio, Tokyo, 142-8555 (JP); OGAWA, Yoshio, Tokyo, 142-8555 (JP); ISHII, Hikaru, Tokyo, 104-0031 (JP)
(74) Representative: Pfenning, Meinig & Partner mbB

(57) **Abstract**

A detection method of detecting CTCs as target cells contained in a test sample, the method includes a concentration step of concentrating the target cells contained in the test sample, a labeling step of labeling the target cells and other components by bringing the concentrated test sample into contact with first labeling antibodies in which antibodies specifically binding to antigens, which exclude antigens expressing in epithelial cells and specifically express in the target cells, are labeled by a first labeling substance, and into contact with second labeling antibodies in which antibodies binding to antigens expressing in the other components excluding the target cells are labeled by a second labeling substance, and a detection step of detecting, from the test sample, cells that are labeled by the first labeling antibodies and are not labeled by the second labeling antibodies as the target cells.

## Description

### BACKGROUND

The present disclosure relates to a detection method and a detection device of circulating tumor cells.

Recently, a "Liquid Biopsy" using, for example, blood sampled from a cancer patient is spotlighted instead of a tissue biopsy that samples cancer tissues from a cancer patient. As this "Liquid Biopsy", a Circulating Tumor Cell inspection (CTC inspection) that detects Circulating Tumor Cells (hereinafter, CTCs) in peripheral blood is considered to be important. The CTCs are released from primary tumor tissues or metastatic tumor tissues, and circulate in blood. The CTCs are considered to have a metastatic potential to other organs. Measuring the number of CTCs in blood with the CTC inspection is thus an effective inspection method by which it can be expected to diagnose a cancer, to diagnose prognosis, to determine a therapeutic effect, to early detect a metastatic cancer, and to understand a condition of a disease (*see,* for example, Vaidyanathan R, Soon RH, Zhang P, Jiang K, Lim CT, "Cancer diagnosis: from tumor to liquid biopsy and beyond.", Lab Chip. 2018 Dec 18;19(1):11-34. doi: 10.1039/c81c00684a).

As a device for the CTC inspection, CellSearch® system, which is an only system approved by a Food and Drug Administration (FDA), is known. This device is configured to specifically separate and extract CTCs from blood with magnetic beads to which antibodies against Epithelial Cell Adhesion Molecules (hereinafter, EpCAMs, CD326) as epithelial cell adhesion molecules are bound, to react the separated and extracted CTCs with fluorescence-labeled cytokeratin, and to perform nuclear staining. In order to distinguish mixed white blood cells and the CTCs, fluorescence-labeled CD45 antibodies are used. After that, a fluorescence image is obtained by irradiating laser beams to the cells floated with a magnetic field. An inspector determines whether the cells are the CTCs or not based on the fluorescence image. In this prior art, CD45 positive cells are eliminated as the white blood cells, and cells that express the EpCAMs and the cytokeratin are detected as the CTCs (*see,* for example, JP4409096B).

However, it is known that the CTCs derived from epithelial cancer cells may cause Epithelial Mesenchymal Transition (hereinafter, EMT), and the expression of the EpCAMs is thereby lowered or disappeared. Accordingly, in the prior art using the EpCAM antibodies, the CTCs which cause the EMT phenomenon cannot be detected, and may be missed.

### SUMMARY

The present disclosure has been made in view of the above circumstances, and an object of the present disclosure is to provide a detection method and a detection device capable of detecting CTCs with high accuracy.

To achieve the above object, one aspect of the present disclosure provides a detection method of detecting CTCs as target cells contained in a test sample. The method includes: a concentration step of concentrating the target cells contained in the test sample; a labeling step of labeling the target cells and other components by bringing the concentrated test sample into contact with first labeling antibodies in which antibodies specifically binding to antigens, which exclude antigens expressing in epithelial cells and specifically express in the target cells, are labeled by a first labeling substance, and into contact with second labeling antibodies in which antibodies binding to antigens expressing in the other components excluding the target cells are labeled by a second labeling substance; and a detection step of detecting, from the test sample, cells that are labeled by the first labeling antibodies and are not labeled by the second labeling antibodies as the target cells.

Another aspect of the present disclosure provides a detection device that detects CTCs as target cells contained in a test sample. The device includes: a concentration part configured to concentrate the target cells contained in the test sample; a detection part configured to detect the target cells that are labeled by first labeling antibodies and are not labeled by second labeling antibodies from the test sample in which the target cells and other components are labeled by bringing the concentrated test sample into contact with the first labeling antibodies in which antibodies specifically binding to antigens, which exclude antigens expressing in epithelial cells and specifically express in the target cells, are labeled by a first labeling substance, and into contact with the second labeling antibodies in which antibodies binding to antigens expressing in the other component excluding the target cells are labeled by a second labeling substance; and a control part configured to create a display image regarding the target cells based on a detection result by the detection part and predetermined gating information and to display the created image on a display part.

### BRIEF DESCRIPTION OF DRAWINGS

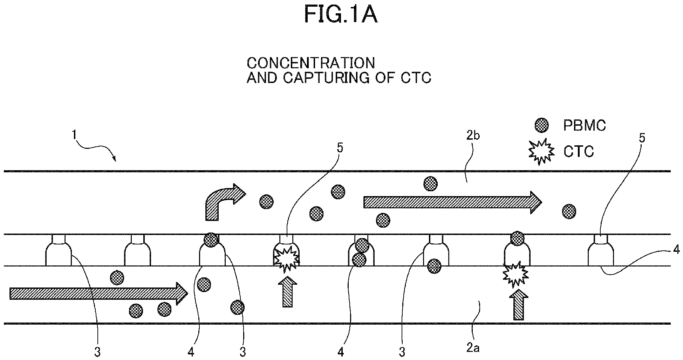

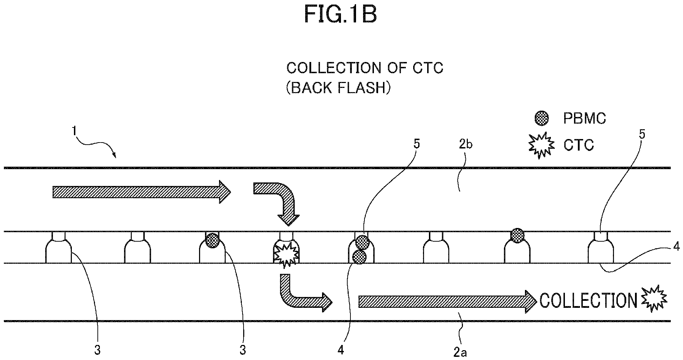

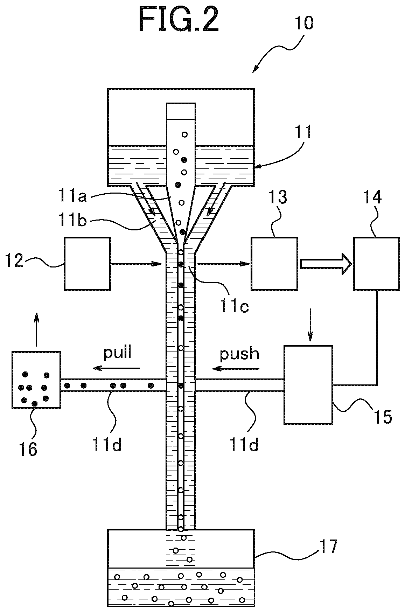

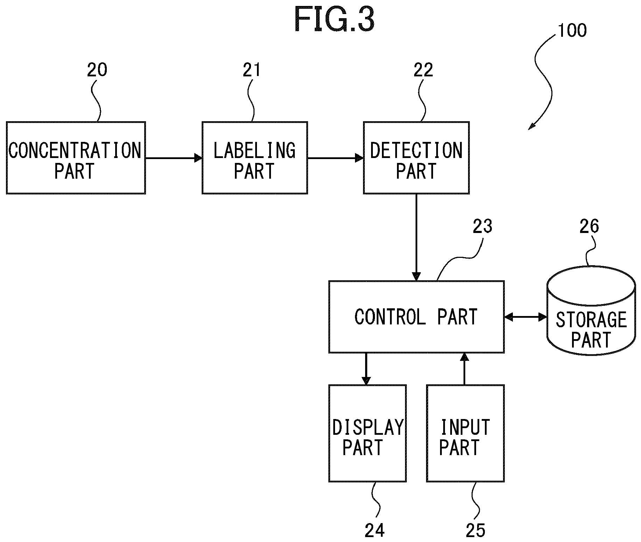

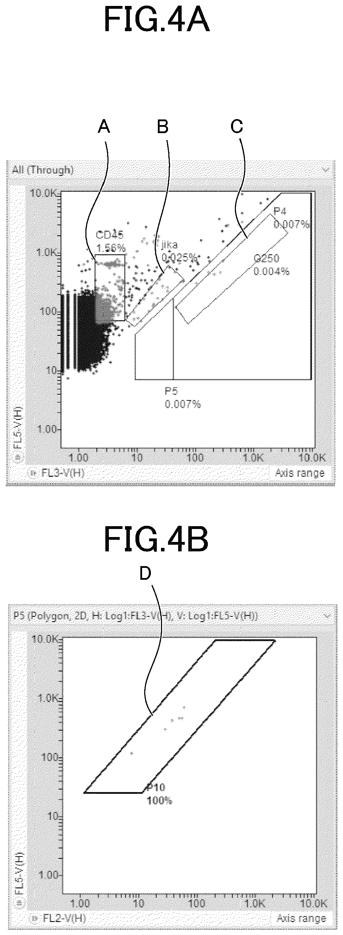

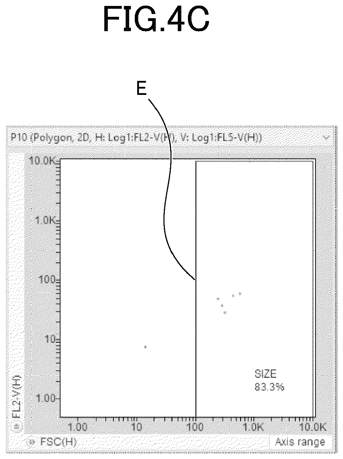

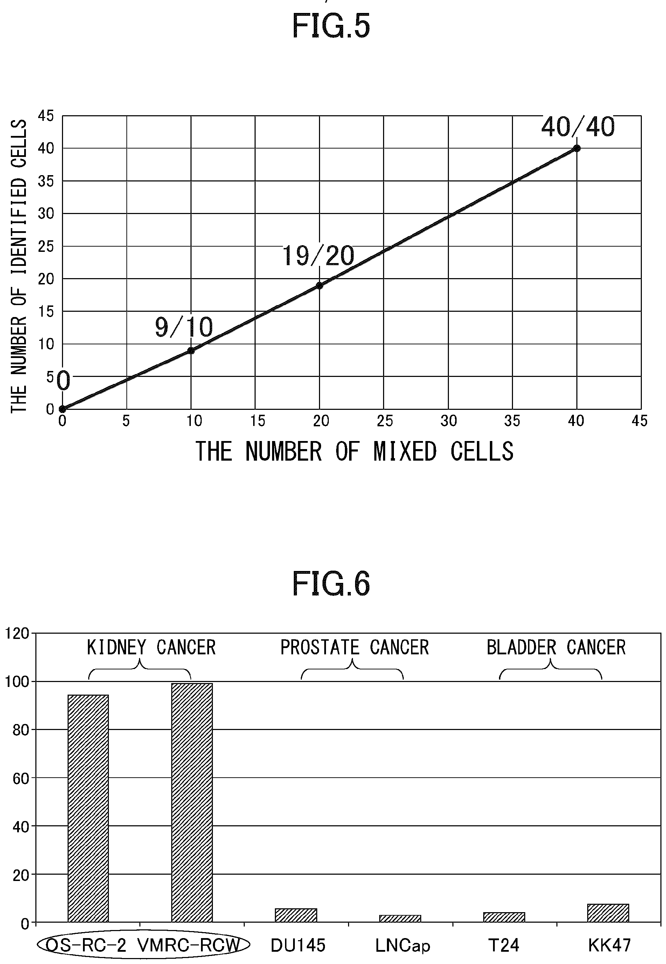

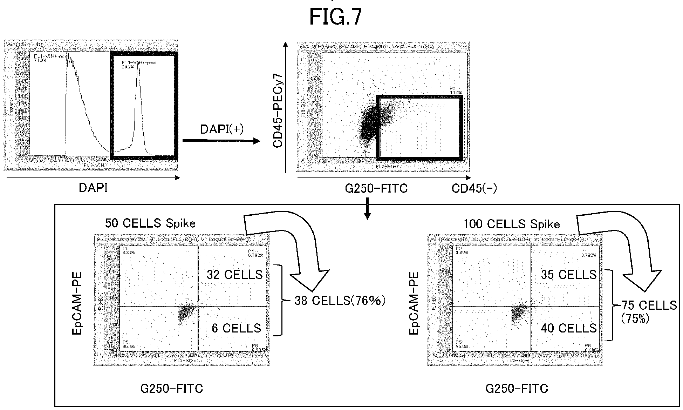

Figs. 1A, 1B are explanation views explaining a concentration process in a detection method of CTCs according to an embodiment; Fig. 1A illustrates that CTCs are concentrated and captured; and Fig. 1B illustrates that the CTCs are collected. Fig. 2 is a view explaining a detection step in the detection method of the CTCs according to the embodiment, and illustrating one example of a detector that performs the detection step. Fig. 3 is a diagram illustrating a schematic configuration of the detection device of the CTCs, which performs the detection method of the CTCs according to the embodiment. Figs. 4A, 4B, 4C show two-dimensional plots based on detection results of kidney cancer CTCs in Example 1; Fig. 4A shows a two-dimensional plot of all detected cells; Fig. 4B shows a two-dimensional plot of the kidney cancer CTCs narrowed by gating; and Fig. 4C shows a two-dimensional plot with a size of a cell as a parameter of an X axis. Fig. 5 is a graph showing an identification rate of the kidney cancer CTCs in Examples 1, 2, 3. Fig. 6 is a histogram showing a result of antigen-antibody reaction of each cancer cell line and an anti-G250 antibody in Experimental Example 1. Fig. 7 is a two-dimensional plot showing an identification result of the kidney cancer CTCs in Experimental Example 2.

### DETAILED DESCRIPTION

With respect to the use of plural and/or singular terms herein, those having skill in the art can translate from the plural to the singular and/or from the singular to the plural as is appropriate to the context and/or application. The various singular/plural permutations may be expressly set forth herein for sake of clarity.

Hereinafter, an embodiment is described. A detection method of CTCs according to the present embodiment is a detection method of detecting the CTCs as target cells contained in a sample, and includes at least following steps.

(1) A concentration step of concentrating the target cells contained in the test sample. (2) A labeling step of labeling the target cells and other components by bringing the concentrated test sample into contact with first labeling antibodies in which antibodies specifically binding to antigens, which exclude antigens expressing in epithelial cells and specifically express in the target cells, are labeled by a first labeling substance and with second labeling antibodies in which antibodies binding to surface antigens expressing in other components excluding the target cells are labeled by a second labeling substance. (3) A detection step of detecting, as the target cells, cells that are labeled by the first labeling antibodies and are not labeled by the second labeling antibodies from the test sample.

The test sample is not limited as long as it is sampled from a cancer patient or a patient with a suspected cancer. Any test sample which may contain the CTCs can be used. More specifically, the test sample is a vivo-derived liquid sample which may contain the CTCs, for example, blood, urine, lymph, tissue fluid, cerebrosphinal fluid, ascite, and pleural effusion. Peripheral blood is suitable among these because it can be easily sampled.

The test sample may be the above described vivo-derived liquid sample, a sample in which the above liquid sample is diluted by saline, or a sample to which an additive is added.

The CTCs as the target cells are cancer cells that are detected at extremely low concentration in blood of a cancer patient. Although the CTCs detected by the detection method of the CTCs of the present embodiment are not limited, the detection method is suitable for detecting the CTCs derived from an epithelial cancer.

The epithelial cancer includes, for example, a bladder cancer, breast cancer, colorectal cancer, rectal cancer, kidney cancer, liver cancer, lung cancer, small cell lung cancer, esophageal cancer, gallbladder cancer, ovarian cancer, pancreatic cancer, gastric cancer, cervical cancer, thyroid cancer, prostate cancer, epidermoid cancer, skin cancer, duodental cancer, vaginal cancer, and brain cancer.

As the detection method of the CTCs in this embodiment does not use EpCAM antibodies as targets, even the CTCs in which the expression of the EpCAMs is lowered or disappeared due to the EMT phenomenon can be detected with high accuracy. Among these epithelial cancers, the detection method is especially suitable for detecting the CTCs derived from, for example, a kidney cancer (clear cell carcinoma) and an urothelial carcinoma (bladder cancer, ureteric cancer, and renal pelvis cancer) for which an effective tumor marker has not existed yet, and is best suitable for detecting the CTCs derived from a kidney cancer.

"Other components" excluding the target cells, which are contained in the test sample, are meant to be cells excluding the CTCs, and are also referred to as contaminant cells or impurity cells. More specifically, "other components" include, for example, Peripheral Blood Mononuclear Cells (PBMCs) such as white blood cells (eosinophil, neutrophil, basophil, monocyte, lymphocyte), and red blood cells. As most of the PBMCs are eliminated by the concentration step and the red blood cells are also eliminated by the concentration step and hemolysis, "other components" contained in the test sample after the concentration mainly include residual PBMCs.

Hereinafter, for example, each step in the detection method of the CTCs, and antibodies and labeling substances for use in each step of the present embodiment are described in details with reference to the drawings.

### (1) Concentration Step

The concentration step is a step of concentrating the CTCs as the target cells contained in the test sample by separating the CTCs from other components. The concentration step is not specifically limited, but a microchannel device method is suitable for the concentration step. The microchannel device method is a method of concentrating the target cells by separating the target cells based on a difference in size of cells from other components with a microchannel device (chip).

As illustrated in Figs. 1A, 1B, a microchannel device 1 includes at least first and second channels 2a, 2b which are layered to each other. The test sample flows in the first and second channels 2a, 2b. The first and second channels 2a, 2b have at least two-layer structure. A plurality of communication paths (tunnel) 3 which communicate the first and second channels 2a, 2b each other are provided between the first and second channels 2a, 2b. The communication path 3 includes an entrance 4 on the upstream side and an exit 5 on the downstream side. The entrance 4 has a size (diameter) larger than a size of the target cell and other components, and the exit 5 has a size (diameter) smaller than the size of the target cell and larger than the size of other components. More specifically, the size (diameter) of the entrance 4 is set to about 25 µm and the size (diameter) of the exit 5 is set to about 8 µm. About fifty thousand communication paths 3 are provided for the single microchannel device 1.

In the above-described microchannel device 1, the test sample L flows from the first channel 2a to the second channel 2b by applying negative pressure from the downstream of the second channel 2b with a syringe while supplying the test sample L into the first channel 2a from the upstream thereof. The test sample L supplied to the first channel 2a thereby flows downstream in the first channel 2a, and flows in the second channel 2b from the first channel 2a through the communication paths 3. In this case, only other components such as the red blood cells and the PBMCs having a size smaller than the size of the exit 5 flow in the second channel 2b through the communication paths 3. In addition, the hemolysis of the red blood cells may be eliminated with known chemical.

On the other hand, the CTC having a size larger than the size of the exit 5 cannot pass through the exit 5, and is captured in the communication path 3, as illustrated in Fig. 1A. Next, after the supply of the test sample L is completed, as illustrated in Fig. 1B, the CTCs can be collected by flowing (Back Flash) the test sample L from the second channel 2b to the first channel 2a. By separating the CTCs and other components based on the difference in size of the cells as described above, the CTCs can be physically concentrated with high efficiency without depending on antigen and antibody response.

A concentration device that performs the concentration step is not specifically limited. For example, Celsee® is suitably used as the concentration device using the above-described microchannel device 1. The CTCs can be thereby simply, efficiently, and rapidly concentrated.

### (2) Labeling Step

The labeling step is a step of labeling (marking) each cell by bringing other components (PBMCs) and the target cells (CTCs) of the concentrated test sample into contact with first labeling antibodies and second labeling antibodies. With this step, the CTCs as the target cells are labeled by the first labeling antibodies and the PBMCs as other components excluding the target cells are labeled by the second labeling antibodies.

A known method can be used for the labeling step. The labeling step includes, for example, a first labeling step of adding the first labeling antibodies to the test sample and incubating the test sample at about 4□ to a room temperature for several minutes to several hours, and a second step of adding the second labeling antibodies to the test sample and incubating the test sample at about 4□ to a room temperature for serval minutes to several hours. Alternatively, the first labeling step and the second labeling step may be simultaneously performed or sequentially performed. When the first labeling step and the second labeling step are sequentially performed, one of the first labeling step and the second labeling step may be performed before the other.

### Antigen Specifically Expressing in Target Cell

An antigen specifically expressing in the CTCs as the target cells is an antigen excluding an antigen expressing in epithelial cells, in order to avoid the deterioration of the CTC detection due to the EMT phenomenon. The antigen expressing in the epithelial cells is an antigen excluding the EpCAM, cytokeratin, E-cadherin, vimentin, and the like. The antigen as the target in this embodiment is not specifically limited as long as it is an antigen excluding the EpCAM antigen, and the like. It may be an antigen specifically expressing in each cancer. More specifically, for example, when the CTCs are circulating kidney cancer cells, a G250 antigen specifically expressing in the kidney cancer is most suitable.

### Surface Antigen Expressing in Other Components

Most of other components excluding the CTCs are eliminated by the concentration step. The residual other components are PBMCs containing white blood cells. The antigen as the target expressing in the PBMCs is not limited, but preferably includes, for example, CD2, CD3, CD4, CD5, CD8, CD10, CD11b, CD14, CD15, CD16, CD19, CD20, CD24, CD25, CD27, CD29, CD33, CD36, CD38, CD41, CD45, CD45RA, CD45RO, CD56, CD66b, CD66e, CD69, and CD124. Among these, the CD45 is most suitable because it exists in most of the PBMCs.

### First Labeling Antibody and Second Labeling Antibody

The first labeling antibody is labeled (marked) by the first labeling substance, and includes an antibody specifically binding to the above "antigen specifically expressing in target cell". The antibody is an antibody against an antigen existing in the CTCs but not in the PBMCs. For example, when the CTCs are the circulating kidney cancer cells, the anti-G250 antibody specifically binding to the G250 antigen that exists in the kidney cancer is suitable.

The second labeling antibody is labeled (marked) by the second labeling substance, and includes an antibody binding to the surface antigen expressing in the above "other components". The antibody is an antibody binding to an antigen existing in the PBMCs but not in the CTCs. The above described antibody binding to the surface antigen of the PBMC is suitably used. As the most suitable antigen for identifying the PBMC is the CD45 among these, the anti-CD45 antibody is most suitable as the antibody.

The first labeling antibody and the second labeling antibody may be a polyclonal antibody, a monoclonal antibody, or a recombinant antibody. The first labeling antibody and the second labeling antibody may be, for example, an antibody fragment including an antigen binding site of these antibodies. The antibody fragment includes, for example, F (ab')2, Fab', Fab, and Fv.

The first labeling substance of the first labeling antibody and the second labeling substance of the second labeling antibody are not specifically limited, and known labeling substances are used for these substances. These labeling substances suitably include, for example, fluorescent dye, fluorescent protein, and luciferin.

The fluorescent dye suitably includes, for example, Alexa Fluor series (Alexa Fluor 488, Alexa Fluor 647), Brilliant Violet series (BV421, BV570), BODIPY series (BODIPY FL, BODIPY TR), Cy3, Cy5, PE-Cy7, FITC (fluorescein isothiocyanate), PE, PerCP, and coumarin fluorescent dye. The fluorescent protein includes, for example, phycocyanin, allophycocyanin, phycoerythrin, and phycoerythrocyanin. The luciferin includes, for example, luciferase, peroxidase, and alkaline phosphatase.

### (3) Detection Step

The detection step is a step of detecting, as the target cells (CTCs), cells that are labeled by the first labeling antibodies and are not labeled by the second labeling antibodies from the cells contained in the test sample labeled by the above labeling step.

Although the detection step is not specifically limited, it is desirable to detect the cells with a flow cytometry based on the fluorescent or the emission signal of the first labeling substance and the second labeling substance because it can detect the cells and also isolate the cells.

The flow cytometry is a technique that flows cells in line and counts the number of cells with a spectroscopic method. For example, the number of target cells are counted by irradiating the cells labeled by the fluorescence or the luciferin with laser beams, and thereby detecting the fluorescence or the emission signals from the cells with a detector such as a photodiode. The detection result with the detector can be loaded into a computer to create and display a two-dimensional plot. The presence of the target cells and the number of target cells can be thereby easily confirmed.

As a method of separating the target cells (cell sorting) detected with the flow cytometry, a method (Jet in Air method) of separating the target cells by controlling a flow direction of electric charged droplets containing the target cells may be used. However, a Flow Shift method is preferably used. The target cells can be detected and separated with high accuracy by using the Flow Shift method. The Flow Shift method is a method of separating the target cells flowing in the microchannel under air pressure control.

The detection step with the flow cytometry and the cell sorting by the Flow Shift method is schematically described with reference to Fig. 2. Fig. 2 is a view illustrating the schematic configuration of the detector that performs the detection step. A detector 10 operates as a flow cytometer and as a cell sorter. As illustrated in Fig. 2, the detector 10 includes a flow cell 11, an irradiation part 12 having a laser light source, a detection part 13 having a photodiode, a pressurizing part 15 having an electromagnetic valve and a pump, a control part 14 that controls the driving of the pressurizing part 15, and a sorting reservoir 16 in which target cells are collected, and a waste liquid reservoir 17 in which waste liquid is accumulated. The flow cell 11 is a microchannel chip having a cross shaped microchannel. The microchannel includes a channel 11a for introducing a test sample, a channel 11b for introducing sheath liquid, a joining channel 11c, and a branching channel 11d that is provided on the downstream of the joining channel 11c and intersects with the joining channel 11c at a right angle.

In the detector 10, the test sample flowing in the channel 11a and the sheath liquid flowing in the flow channel 11b join at the joining channel 11c. A significantly thin flow of the test sample can be thereby created by hydrodynamic focusing. The cells can thereby flow in a line.

The irradiation part 12 irradiates laser beams to the test sample flowing in the joining channel 11c. The fluorescence or the scattering light is thereby generated from the cells labeled by the first and second labeling substances. The detection part 13 detects the fluorescence or the scattering light, and identifies the target cells (CTCs) and other components (PBMCs) based on the intensity of these signals to detect the target cells.

The detection result by the detection part 13 is sent to the control part 14. The control part 14 displays, on a display such as a monitor, the detection result, a histogram, a two-dimensional plot, and a three-dimensional plot according to a purpose. At a moment that the target cells pass through the intersection part of the joining channel 11c and the branching channel 11d, the control part 14 operates the pump by controlling the electromagnetic valve, and applies positive (Push) and negative (Pull) pressures respectively from both sides of the branching channel 11d. The target cells are thereby moved to the branching channel 11d, and are collected in the sorting reservoir 16. On the other hand, the other components excluding the target cells flow down the joining channel 11c, and are collected in the waste liquid reservoir 17. Such a detector 10 can rapidly and easily detect and separate the target cells without depending on the EpCAMs.

As the above-described detector 10, for example, On-Chip Sort® manufactured by On-Chip Biotechnologies Co., Ltd is preferably used. By using On-Chip Sort®, various effects can be achieved, for example, the target cells can be detected with high accuracy, the damage to the cells can be controlled, the separation can be performed under a non-aseptic environment, the separation can be performed even in culture solution, a small amount of sample can be used, and the device can be installed in a clean bench.

### Another Step

The detection step of the CTCs in this embodiment may include a gating step of detecting reference target cells with the flow cytometry by using a reference sample in which the known number of reference target cells are mixed, and of previously performing the gating based on the detection result.

In this gating step, at first, the reference sample is obtained, as the reference target cells, by spiking peripheral blood sampled from a healthy body with the known number of incubated cancer cells. After the above-described concentration step (1) and labeling step (2) are performed to the obtained reference sample, the cells that are labeled by the first labeling antibodies and are not labeled by the second labeling antibodies are detected with the flow cytometry by the same step as the detection step (3). The gating is performed by designating a region where the reference target cells exist based on the detection result (histogram, two-dimensional plot).

When the CTCs are detected from the test sample, the target cells are detected by the detection step (3) with the flow cytometry to the test sample based on the gating obtained by the gating step. The target cells can be further reliably and effectively detected.

As described above, in the detection method of the CTCs according to the present embodiment, other components (for example, PBMCs) excluding the target cells can be effectively eliminated by concentrating the target cells (CTCs) contained in the test sample by the concentration step. The target cells can be labeled by the first labeling antibodies, and other components excluding the target cells can be labeled by the second labeling antibodies by the labeling step. By differentiating the first labeling substance and the second labeling substance, the target cells and other components excluding the target cells can be distinguished. In the next step, the cells that are labeled by the first labeling antibodies and are not labeled by the second labeling antibodies can be thereby easily and reliably detected as the target cells from the target sample.

The antigens excluding the antigens expressing in the epithelioid cells such as EpCAM antigens and specifically expressing in the target cells are targeted as the antigens of the target cells. The first labeling antibodies specifically binding to such antigens are bound to such antigens. Accordingly, in the detection method of the CTCs in the present embodiment, even the cancer cells causing the EMT can be detected with high accuracy without depending on the EpCAMs. As a result, the detection accuracy of the CTCs can be further improved.

The detection method of the CTCs according to the present embodiment is specifically effective for detecting the kidney cancer CTCs. The anti-G250 antibodies are used as the first labeling antibodies. The anti-G250 antibodies specifically bind to the G250 antigens and specifically expressing in the kidney cancer CTCs are labeled by the first labeling substance. The kidney cancer CTCs can be thereby detected with high accuracy without depending on the EpCAMs. The anti-CD45 antibodies labeled by the second labeling substance are used as the second labeling antibodies to eliminate the PBMCs contained in the test sample. The cells of the G250 positive and the CD45 negative can be thereby easily detected with high accuracy as the kidney cancer CTCs.

Next, an embodiment of a detection device of the CTCs that performs the detection method of the CTCs according to the present embodiment is described with reference to the drawings. Fig. 3 is a schematic view illustrating an entire configuration of a CTC detection device 100 as one embodiment of the detection device of the CTCs.

As illustrated in Fig. 3, the CTC detection device 100 according to the present embodiment includes a concentration part 20, a labeling part 21, a detection part 22, a control part 23, a display part 24, an input part 25, and a storage part 26.

The concentration part 20 performs the concentration step of concentrating the target cells contained in the test sample. For example, the microchannel device 1 illustrated in Fig. 1 can be used as the concentration part 20.

The labeling part 21 performs the labeling step of labeling the test sample by contacting the first labeling antibodies and the second labeling antibodies with the test sample. The labeling part 21 may be configured to automatically or manually put the first labeling antibodies and the second labeling antibodies in a flask in which the test sample is housed.

The detection part 22 performs the detection step of detecting the target cells from the test sample contacted by the first and second labeling antibodies. For example, the detector 10 illustrated in Fig. 2 can be used as the detection part 22.

The control part 23 includes a CPU. The control part 23 controls an entire operation of the CTC detection device 100 by developing a program stored in the storage part 26 on a RAM, for example. The control part 23 creates a display image (for example, histogram, two-dimensional plot) regarding the target cells based on the detection result of the detection part 22 and the gating information stored in the storage part 26 to be displayed on the display part 24.

The control part 23 may be provided in an information processing device such as a personal computer including the display part 24 and the input part 25. When the detector 10 as illustrated in Fig 2 as the detection part 22 is used, the control part 14 of the detector 10 may operate as the control part 23.

The display part 24 displays the display image such as a histogram. The input part 25 receives the input of the gating information. The display part 24 uses a touch panel display such as a liquid crystal display and an organic EL. The display part 24 is provided with the touch panel input part 25 that is superimposed on a display surface on which the image is displayed. The input part 25 additionally includes a mouse and a keyboard.

The storage part 26 includes, for example, a Read Only Memory (ROM), a Random Access Memory (RAM), a flash memory, and a hard disc. The storage part 26 stores information such as various programs and parameters required for operating the CTC detection device 100. The storage part 26 stores, for example, the detection results of the CTCs and the gating information.

In the CTC detection device 100 configured as described above, the test sample is concentrated by the concentration part 20, and the first labeling antibodies and the second labeling antibodies contact the test sample in the labeling part 21. Next, the labeled test sample is supplied to the detection part 22. The CTCs are thereby detected and separated by the detection part 22. The control part 23 creates a predetermined histogram based on the detection results of the detection part 22, and displays the predetermined histogram on the display part 24. In this case, the control part 23 may display only the CTC data by gating each of the detected cells. The number of the detected CTCs can be thereby further clearly obtained.

The gating information may be predetermined gating information stored in the storage part 26. A user may designate a region by clicking the mouse or touching the touch panel of the input part 25 while visually reorganizing the display image, and may create the histogram by extracting only the cell group existing in that region with the control part 23 to be displayed.

When the gating information is predetermined, the reference target cells may be detected with the CTC detection device 100 by using the reference sample in which the known number of reference target cells are mixed, and the gating information may be determined based on the detection result. More specifically, the reference sample is concentrated by the concentration part 20, and the test sample contacts the first and second labeling antibodies by the labeling part 21. The cells that are labeled by the first labeling antibodies and are not labeled by the second labeling antibodies are detected as the reference target cells from the reference sample with the detection part 22. Following the detection result, the control part 23 generates the display image to be displayed on the display part 24. When a user designates an appropriate region containing the target cells by the input part 25 while visually recognizing the display image, the control part 23 obtains the coordinate of the designated region to be stored in the storage part 26 as the gating information. The control part 23 may automatically create the gating information based on the detection result to be stored in the storage part 26.

When the target cells are detected from the test sample by previously obtaining the gating information with the reference sample, and storing the gating information in the storage part 26 as described above, the histogram regarding only the target cells can be automatically and rapidly displayed with high accuracy to be presented to the user.

The CTC detection device 100 configured as described above may be configured by the combination of Celsee® and On-Chip Sort® or one device including these operations.

### Examples

Hereinafter, the present embodiment is described in details with reference to the examples. However, the present embodiment is not limited to the examples.

### Example 1

The CTCs were detected with the detection method of the CTCs according to the present embodiment by using a model sample containing the CTCs as the test sample.

### Adjustment of Test Sample

Peripheral blood of 4 cc was sampled from a healthy person as the test sample. The test sample was adjusted by spiking (mixing) the peripheral blood with the ten kidney cancer CTCs derived from the kidney cancer cells. VMRC-RCW was used for the kidney cancer cells.

### Purification of First Labeling Antibody

Anti-Carbonic Anhydrase 9-PE human (manufactured by Miltenyi Biotec GmbH) was used as the first labeling antibodies (anti-G250 antibodies) that recognize the G250 antigens of the kidney cancer CTCs to be bound.

### Purification of Second Labeling Antibody

PerCP anti-human CD45 (manufactured by BioLegend, Inc) was used as the second labeling antibodies (anti-CD45 antibodies) that recognize the CD45 antigens expressing in the PBM to be bound.

### Concentration of Test Sample

The adjusted test sample was concentrated with Celsee®.

### Labeling of Cell Group

After the condensed 4cc test sample was centrifuged, and suspended in a buffer of 100 µL to 200 µL, 10 µL first labeling antibodies (anti-G250 antibodies) were added and 5 µL second labeling antibodies (anti-CD45 antibodies) were added. The kidney cancer cells and the PBMCs were thereby labeled.

### Detection of Kidney Cancer CTC

The test sample containing the labeled cell group was analyzed with the flow cytometry by using On-Chip Sort®, and the kidney cancer CTCs were detected. Figs. 4A, 4B show the two-dimensional plot based on the detection results. Fig. 4A shows the two-dimensional plot of all of the detected cells. A polygonal region (gate region) A in Fig. 4A shows the anti-CD45 antibody positive cells, namely, the PMBCs. A region B shows the autofluorescence cells. A region C shows the anti-G250 antibody positive cells, namely, the kidney cancer CTCs.

The PBMCs and the autofluorescence cells were eliminated by the gating from the two-dimensional plot in Fig. 4A, and only the kidney cancer CTCs in the region C were narrowed. The two-dimensional plot in Fig. 4B was thereby created. As illustrated in Fig. 4C, the two-dimensional plot with the horizontal axis of a size was created for the cells in the region D shown in Fig. 4B. In Fig. 4C, a rectangular region E shows the kidney cancer CTCs, and an outside of the region E shows components excluding the kidney cancer CTCs (e.g., debris, waste).

According to these Figs. 4B, 4C, nine spiked kidney cancer CTCs were detected out of ten spiked kidney cancer CTCs. According to the detection method and the detection device of the CTCs in the present embodiment, the result of Example 1 shows that the CTCs can be detected with high accuracy.

### Examples 2, 3

The test samples in Examples 2, 3 were adjusted by spiking 4cc peripheral blood of a healthy person with 20 kidney cancer CTCs and 40 kidney cancer CTCs, respectively. The test samples of Examples 2, 3 were concentrated and labeled, and the kidney cancer CTCs were detected by the steps in the same manner as those in Example 1.

Fig. 5 is a graph of an identification rate of the kidney cancer CTCs based on the detection results in Examples 1 to 3. In this graph, "The Number of Mixed Cells" on the X axis shows the number of kidney cancer cells spiked into the peripheral blood and "The Number of Identified Cells" on the Y axis shows the number of kidney cancer CTCs actually detected in each example. According to the present embodiment, as shown in the graph of Fig. 5, the kidney cancer CTCs in the peripheral blood can be detected with high probability such as 90% to 100%.

### Experimental Example 1

A verification experiment was performed for verifying the G250 antigen as an effective marker of a kidney cancer. In this Experimental Example 1, the anti-G250 antibodies contacted kidney cancer cell lines (OS-RC02, VMRC-RCW), prostate cancer cell lines (DU145, LNCap), and bladder cancer cell lines (T24, KK47). Fig. 6 shows the result of the antigen-antibody reaction. The result in Fig. 6 shows that the kidney cancer cell lines were positive to the anti-G250 antibodies with high probability close to 100%. Accordingly, it is apparent that the anti-G250 antibodies are effective for detecting the kidney cancer CTCs.

### Experimental Example 2

As Experimental Example 2, a verification experiment was performed for verifying that even the CTCs causing the EMT can be detected with high accuracy by using the anti-G250 antibodies. In Experimental Example 2, experimental test samples 1, 2 were adjusted by spiking 4cc peripheral blood of a healthy person with 50 VMRC-RCWs and 100 VMRC-RCWs as kidney cancer cell founder lines, respectively.

After hemolyzation, white blood cells were eliminated from the experiment test samples 1, 2 by using magnetic beads coated by the anti-CD45 antibodies, and the kidney cancer CTCs were concentrated. After that, the anti-G250 antibody labeled by FITC and the anti-EpCAM antibodies labeled by PE were respectively reacted with the concentrated kidney cancer CTCs to compare the specificity of the anti-G250 antibodies and the specificity of the anti-EpCAM antibodies against the kidney cancer CTCs. The anti-CD45 antibodies labeled by PE-Cy7 were also reacted with the concentrated kidney cancer CTCs to be distinguished from the PBMCs.

The kidney cancer CTCs were distinguished from the PBMCs by using On-Chip Sort® after the labeling. The anti-CD45 antibody negative and anti-G250 antibody positive cells were counted as the kidney cancer CTCs. Fig. 7 shows the identification results (detection results) of the kidney cancer CTCs. The two-dimensional plot in the upper side in Fig. 7 shows that the kidney cancer CTCs can be detected with high accuracy with the G250 antigens as the targets.

When the anti-CD45 antibody negative and anti-EpCAM antibody positive cells counted as the kidney cancer CTCs in the two-dimensional plot in the lower side in Fig. 7, 32 kidney cancer CTCs out of 50 kidney cancer CTCs in Experimental Example 1 and 35 kidney cancer CTCs out of 100 kidney cancer CTCs in Experimental Example 2 were eliminated. It is assumed that these CTCs were not detected because these CTCs were cells causing the EMT. On the other hand, when the anti-CD45 antibody negative and anti-G250 antibody positive cells were counted as the kidney cancer CTCs, the kidney cancer CTCs were detected with probability of 38 kidney cancer CTCs out of 50 kidney cancer CTCs (76%) in Experimental Example 1 and with probability of 75 kidney cancer CTCs out of 100 kidney cancer CTCs (75%) in Experimental Example 2. Namely, even the cells causing the EMT can be detected with high accuracy.

The above results show that it is significantly effective to use the anti-G250 antibodies with the G250 antigens as the targets for detecting the kidney cancer CTCs.

Although the present invention has been described in terms of exemplary embodiments, it is not limited thereto. It should be appreciated that variations or modifications may be made in the embodiment, examples, experimental examples described by persons skilled in the art without departing from the scope of the present invention as defined by the following claims.

According to the detection method and the detection device of the CTCs in the present disclosure, as the CTCs can be detected with high accuracy, the detection results can be preferably used for diagnosing a cancer, diagnosing prognosis, determining a therapeutic effect, early detecting a metastatic cancer, and understanding a condition of a disease. Patients expected to benefit from an effect of anti-cancer agents and other cancer treatments can be thereby specified, patients who are likely to have a specific side effect can be thereby specified, application and volume of anti-cancer agents can be optimized, and discontinuation of administration of the anti-cancer agents can be appropriately determined. The detection method and the detection device of the present disclosure can be also used as a new biomarker or a test kit, and also can be applied to prediction of an effect of a companion diagnostic agent and a field of a companion diagnostic.

## Claims

1. A detection method of detecting CTCs as target cells contained in a test sample,
the method comprising:
a concentration step of concentrating the target cells contained in the test sample;
a labeling step of labeling the target cells and other components by bringing the concentrated test sample into contact with first labeling antibodies in which antibodies specifically binding to antigens, which exclude antigens expressing in epithelial cells and specifically express in the target cells, are labeled by a first labeling substance, and into contact with second labeling antibodies in which antibodies binding to antigens expressing in the other components excluding the target cells are labeled by a second labeling substance; and
a detection step of detecting, from the test sample, cells that are labeled by the first labeling antibodies and are not labeled by the second labeling antibodies as the target cells.

2. The detection method according to claim 1, wherein
the target cells are kidney cancer CTCs, and
the antibodies of the first labeling antibodies are anti-G250 antibodies specifically binding to G250 antigens specifically expressing in the kidney cancer CTCs.

3. The detection method according to claim 1, wherein
the other components are monocular cells containing white blood cells, and the antibodies of the second labeling antibodies are anti-CD45 antibodies binding to CD45 antigens expressing in surfaces of the monocular cells.

4. The detection method according to claim 1, wherein
the antigens expressing in the epithelial cells are EpCAMs, cytokeratin, E-cadherin, or vimentin.

5. The detection method according to claim 1, wherein
the concentration step is a step of concentrating the target cells by separating the target cells from the other components based on a difference in size of a cell with a microchannel device method,
the microchannel device method includes at least a first channel (2a) and a second channel (2b) that are layered to each other, the test sample flowing in the first and second channels (2a, 2b),
a plurality of communication paths (3) that communicate the first and second channels (2a, 2b) are provided between the first and second channels (2a, 2b),
the test sample flows from the first channel (2a) and the second channel (2b) through the communication paths (3), and
the target cells are concentrated with a microchannel device (2) including an entrance (4) provided in the communication path (3) on the first channel side and an exit (5) provided in the communication path (3) on the second channel side, the entrance (4) having a size larger than a size of the target cell and a size of the other component, and the exit (5) having a size smaller than the size of the target cell and larger than the size of the other component.

6. The detection method according to claim 1, wherein
the detection step detects the target cells with a flow cytometry based on fluorescence or an emission signal of the first labeling substance and the second labeling substance, and separates the target cells detected by a separation method of a Flow Shift method that separates cells flowing in a microchannel with air pressure control.

7. The detection method according to claim 6, comprising:
a gating step of concentrating a reference test sample mixed with a known number of reference target cells, of contacting the reference test sample with the first labeling antibodies and the second labeling antibodies, of detecting, from the reference test sample, cells that are labeled by the first labeling antibodies and are not labeled by the second labeling antibodies as the reference target cells with the flow cytometry, and of gating to contain the detected reference target cells, wherein
in the detection step, the target cells are detected from the test sample with the flow cytometry based on the gating.

8. A detection device that detects CTCs as target cells contained in a test sample,
the device comprising:
a concentration part (20) configured to concentrate the target cells contained in the test sample;
a detection part (22) configured to detect the target cells that are labeled by first labeling antibodies and are not labeled by second labeling antibodies from the test sample in which the target cells and other components are labeled by bringing the concentrated test sample into contact with the first labeling antibodies in which antibodies specifically binding to antigens, which exclude antigens expressing in epithelial cells and specifically express in the target cells, are labeled by a first labeling substance, and into contact with the second labeling antibodies in which antibodies binding to antigens expressing in the other components excluding the target cells are labeled by a second labeling substance; and
a control part (14, 23) configured to create a display image regarding the target cells based on a detection result by the detection part and predetermined gating information and to display the created image on a display part (24).

9. The detection device according to claim 8, comprising:
an input part (25) that receives input of the gating information, wherein
the control part (14, 23) is configured to create the display image to be displayed on the display part (24) based on a detection result of reference target cells that are detected by the detection part (22) from a reference test sample, and to obtain the gating information input by the input part (25) based on the display image to be stored in a storage part (26), the reference test sample in which a known number of the reference target cells is mixed being concentrated and being contacted with the first labeling antibodies and the second labeling antibodies.
